# EUROPEAN PATENT SPECIFICATION

(11) **EP 3 462 938 B1**
(45) Date of publication and mention of the grant of the patent: **18.11.2020**
(21) Application number: 17726239.1
(22) Date of filing: 22.05.2017
(51) Int. Cl.: A24F 47/00, H05B 3/34

(54) **FLUID PERMEABLE HEATER ASSEMBLY FOR AEROSOL-GENERATING SYSTEMS**
FLUIDDURCHLÄSSIGE HEIZUNGSANORDNUNG FÜR AEROSOLERZEUGUNGSSYSTEME
ENSEMBLE DE CHAUFFAGE PERMÉABLE AUX FLUIDES POUR SYSTÈMES DE GÉNÉRATION D'AÉROSOL

(30) Priority: 31.05.2016 EP 16172198
(43) Date of publication of application: 10.04.2019
(73) Proprietor: Philip Morris Products S.A., 2000 Neuchâtel (CH)
(72) Inventor: MIRONOV, Oleg, 1588 Cudrefin (CH); ZINOVIK, Ihar Nikolaevich, 2034 Peseux (CH)
(74) Representative: Spencer, James Michael
(86) International application number: PCT/EP2017/062257
(87) International publication number: WO 2017/207322

(56) References cited:
- WO-A1-2015/117701
- CN-B- 102 425 023
- CN-B- 102 443 954
- DE-U1- 20 111 067

## Description

The invention relates to fluid permeable heater assemblies for aerosol-generating systems. In particular, it relates to flat fluid permeable heater assemblies comprising a flat filament arrangement.

WO 2015/117701 A1 describes a fluid permeable heater assembly for aerosol-generating systems comprises a substrate comprising an opening through the substrate, an electrically conductive substantially flat filament arrangement arranged over the opening, and clamping means mechanically fixing the filament arrangement to the substrate. The clamping means are electrically conductive and serve as electrical contacts for providing a heating current through the filament arrangement as well as for stabilizing the filament arrangement clamped to the substrate .

It would be desirable to have a fluid permeable heater assembly with improved performance. In particular, it would be desirable to have a fluid permeable heater assembly having optimized contact and heating performance.

The present invention is defined in the appended claims

According to the invention, there is provided a fluid permeable heater assembly for aerosol-generating systems. The fluid permeable heater assembly comprises an electrically conductive flat filament arrangement and a first contact point and a second contact point for electrically contacting the flat filament arrangement and connecting the flat filament arrangement to an external power source. A longitudinal axis is defined between the first contact point and the second contact point. In the heater assembly, a center resistance Rc is the electrical resistance between two points situated on the longitudinal axis, one of the two points being situated at a distance from the first contact point equal to 40 percent and the other one of the two points being situated at a distance from the first contact point equal to 60 percent of the distance between the first and the second contact point. A first resistance R1 is an electrical resistance between the first contact point and a point situated on the longitudinal axis at a distance from the first contact point equal to 20 percent of the distance between the first and the second contact point. A second resistance R2 is an electrical resistance between the second contact point and a point situated on the longitudinal axis at a distance from the first contact point equal to 80 percent of the distance between the first and the second contact point. A ratio of the center resistance to the first resistance Rc/R1 is between 2 and 400, and a ratio of the center resistance to the second resistance Rc/R2 is between 2 and 400.

Preferably, the ratio of the center resistance to the first resistance Rc/R1 is between 2 and 300, more preferably between 40 and 200.

Preferably, the ratio of the center resistance to the second resistance Rc/R2 is between 2 and 300, more preferably between 40 and 200.

The heater assembly comprises a total resistance Rt corresponding to the electrical resistance between the first contact point and the second contact point.

Preferably, a ratio of the center resistance to the total resistance Rc/Rt corresponds to at least 0.3 or 0.4 preferably 0.5 or 0.6 or 0.7.

Preferably, a ratio of the first resistance to the total resistance R1/Rt is between 0.005 and 0.125, preferably above 0.01, more preferably between 0.01 and 0.1, even more preferably between 0.05 and 0.1.

Preferably, a ratio of the second resistance to the total resistance R2/Rt is between 0.005 and 0.125, preferably above 0.01, more preferably between 0.01 and 0.1, even more preferably between 0.05 and 0.1.

Preferably, the center resistance Rc corresponds to at least 50 percent of a total electrical resistance Rt of the heater assembly between the first and second contact points. Preferably, the first and second resistance each correspond to a maximum of about 13 percent of the total electrical resistance and to a minimum of about 0.5 percent of the total electrical resistance Rt.

The center resistance Rc may correspond up to about 99 percent of the total resistance Rt. Preferably, the center resistance corresponds to about 80 percent to about 98 percent, more preferably to about 90 percent to about 95 percent of the total resistance Rt. Such high electrical resistance in one selected region of the filament arrangement allows targeted resistive heating of the filaments in this heating region and efficient evaporation of an aerosol-forming fluid to be evaporated.

As a general rule, whenever the term "about" is used in connection with a particular value throughout this application this is to be understood such that the value following the term "about" does not have to be exactly the particular value due to technical considerations. However, the term "about" used in connection with a particular value is always to be understood to include and also to explicitly disclose the particular value following the term "about"

Regions next to and between the first and second contact points comprising the relatively low first and second resistance R1, R2 define electrical contact regions of the heater assembly. The contact regions are designed to not, or not substantially, transform current flowing through the contact regions of the filament arrangement into heat. A central region between the first and second contact point comprising the relatively high center resistance defines a heating region of the heater assembly.

The ratio of electrical resistance between center resistance and first and second resistance in the ranges defined above, in particular, a low electrical resistance close to the first and second contact points corresponding to a maximum of about 13 percent each of the total electrical resistance and at the same time to a minimum of about 0.5 percent of the total electrical resistance has been found to be beneficial to the performance of a heater assembly.

The low electrical resistance close to the contact points is preferably much smaller than the electrical resistance of the heating region. The electrical resistance close to the contact points may also have a defined minimum.

A low electrical resistance close to the contact points may positively influence an electrical contact of the heater assembly compared to, for example, heater assemblies comprising filament arrangements comprising meshes having low mesh densities, for example, like meshes preferred for heating regions of a filament arrangement. In addition, the low electrical resistance provides good transport of a heating current to the more centrally arranged heating region, where heating is desired. On the other hand, having a specific ratio of center resistance to first and second resistance, in particular a minimum electrical resistance in contact regions has the benefit of limiting dissipation of heat from the heating region to the contact regions. By this, heat may be kept in a center surface of a heater assembly where evaporation takes place. Overall power consumption of a heater or a respective aerosol-generating device may be limited. In addition, any possibly present overmoulding material in contact regions, typically a polymer material, is less affected by heat.

This variability in resistance distribution in a heater assembly, for example by selection of specific material, sizes or structure of a heating region and contact regions allow to vary, in particular enlarge, a total size of a filament arrangement, however, without varying too much, in particular enlarging a heating region. This may be required or desired in order to not impose excessive demands to a power system of an aerosol-generating device.

The heater assembly according to the invention may have a total resistance Rt between about 0.5 Ohm and about 4 Ohm, more preferably between about 0.8 Ohm and about 3 Ohm, even more preferably about 2.5 Ohm.

Preferably, the center resistance Rc is higher than about 0.5 Ohm, more preferably higher than about 1 Ohm, even more preferably about about 2 Ohm.

Preferably, the first resistance R1 is lower than about 100 mOhm, more preferably lower than about 50 mOhm, for example the resistance is between about 5 mOhm and about 25 mOhm. Preferably, the first resistance is higher than about 3 mOhm, more preferably higher than about 5 mOhm.

Preferably, the second resistance R2 is lower than about 100 mOhm, more preferably lower than about 50 mOhm, for example the resistance is between about 5 mOhm and about 25 mOhm. Preferably, the second resistance is higher than about 3 mOhm, more preferably higher than about 5 mOhm.

Throughout this application, whenever a value is mentioned, this is to be understood such that the value is explicitly disclosed. However, a value is also to be understood as not having to be exactly the particular value due to technical considerations.

Resistance of the heater assembly according to the invention is different to, for example, prior art heater assemblies comprising mesh filaments, where a homogenous mesh with a same mesh density over the entire filament arrangement is mounted to a heater assembly or where a filament arrangement is comprised of a mesh with two side metal plates as contacts. The resistance in contact regions is higher than when using metal plates as contacts but may be the same or higher in a heating region, depending on, for example, a material or a filament construction used for the central heating region.

Due to the defined low electrical resistance close to the contact points, a resistance over the heater assembly may be optimized in view of contacting and heating of the filament arrangement as well as in view of assembly and use of a heater assembly.

A value of a center resistance of a heater assembly may be defined and chosen according to a desired evaporation result or, for example, according to parameters of a heater assembly or of an aerosol-generating device the heater assembly is to be used with. For example, the value of the center resistance may be chosen according to a liquid to be evaporated (viscosity, evaporation temperature, amount of evaporated substance etc.).

Preferably, the arrangement and electrical resistance of a heating region is provided and adapted for a liquid to be efficiently heated and evaporated by the filaments of a center surface of the filament arrangement.

Preferably, the arrangement and electrical resistance of contact regions of a heater assembly or of a first and a second side surface of a filament arrangement is provided and adapted for good electrical contact of the filament arrangement to an external power source. The contact regions are also adapted for an optimal interplay with the heating region or a center surface of a filament arrangement, respectively.

The heater assembly according to the invention may further comprise a first transition resistance R1tp corresponding to the electrical resistance between two points situated on the longitudinal axis, one of the two points being situated at a distance from the first contact point equal to 20 percent and the other one of the two points being situated at a distance from the first contact point equal to 40 percent of the distance between the first and the second contact point. The heater assembly may further comprise a second transition resistance R2tp corresponding to the electrical resistance between two points situated on the longitudinal axis, one of the two points being situated at a distance from the first contact point equal to 60 percent and the other one of the two points being situated at a distance from the first contact point equal to 80 percent of the distance between the first and the second contact point. A ratio of the first transition resistance to the first resistance R1tp/R1 is between 1.1 and 400, a ratio of the second transition resistance to the second resistance R2tp/R2 is between 1.1 and 400, a ratio of the center resistance to the first transition resistance Rc/R1tp is between 1.1 and 400, and a ratio of the center resistance to the second transition resistance Rc/R2tp is between 1.1 and 400.

Preferably, the ratios R1tp/R1, R2tp/R2, Rc/R1tp and Rc/R2tp are between 2 and 300, more preferably between 40 and 200.

A first transition surface of the filament arrangement comprising the first transition resistance R1tp is arranged between the first side surface and the center surface of the filament arrangement or heater assembly, respectively. A second transition surface of the filament arrangement comprising the second transition resistance R2tp is arranged between the second side surface and the center surface. Each transition surface comprises an electrical resistance substantially ranging from the first or second resistance of the corresponding first or second side surface to the center resistance of the center surface.

By the provision of a transition electrical resistance, for example by the provision of a gradient in the electrical resistance, a smooth transition of power distribution over the heater assembly and respective heating may be achieved.

Preferably, a transition resistance is closer to the first or second resistance than the center resistance.

The first and second transition resistance extend over 20 percent of the longitudinal axis between the first and the second contact point of the heater assembly.

The term 'flat' heater assembly or 'flat' filament arrangement is used throughout the specification to refer to a filament arrangement or a flat heater assembly that is in the form of a substantially two dimensional topological manifold. Thus, the flat filament arrangement and flat heater assembly extend in two dimensions along a surface substantially more than in a third dimension. In particular, the dimensions of the flat filament arrangement in the two dimensions within the surface is at least 5 times larger than in the third dimension, normal to the surface. An example of a flat filament arrangement and a flat heater assembly is a structure between two substantially parallel imaginary surfaces, wherein the distance between these two imaginary surfaces is substantially smaller than the extension within the surfaces. In some preferred embodiments, the flat filament arrangement is planar and the flat heater assembly is substantially planar. In other embodiments, the flat filament arrangement and the flat heater assembly is curved along one or more dimensions, for example forming a dome shape or bridge shape.

A flat filament arrangement is preferably used in a flat heating element, which can be easily handled during manufacture and provides for a robust construction.

The term 'filament' is used throughout the specification to refer to an electrical path arranged between two electrical contacts. A filament may arbitrarily branch off and diverge into several paths or filaments, respectively, or may converge from several electrical paths into one path. A filament may have a round, square, flat or any other form of cross-section. A filament may be arranged in a straight or curved manner.

The term 'filament arrangement' is used throughout the specification to refer to an arrangement of one or preferably a plurality of filaments. The filament arrangement may be an array of filaments, for example arranged parallel to each other. Preferably, the filaments may form a mesh. The mesh may be woven or non-woven. Preferably, the filament arrangement has a thickness of between about 0.5 micrometers and about 500 micrometers. The filament arrangement may, for example, be in the form of an array of parallel or crosswise electrically conductive filaments. The filament may be integrally formed with electrical contacts, for example formed from an electrically conductive foil, for example, stainless steel foil, that is etched to define the filaments or openings in a center surface as well as in side surfaces.

A center surface of a filament arrangement is always arranged in between a first and a second side surface of the filament arrangement. Preferably, the center surface is arranged in the geometric middle between the first and the second side surfaces. In a filament arrangement having a longitudinal extension larger than a transverse extension such as, for example a rectangular shaped filament arrangement, the center surface as well as the side surfaces may also have a longitudinal or rectangular shape.

An electrical resistance in the first and the second side surface of a filament arrangement may be selected according to a heating regime through the filament arrangement or according to the way of contacting the filament arrangement to a heater substrate or contacting the heater assembly.

The first and second resistance may be distributed homogenously over each of the two side surfaces.

The first and second resistance may be distributed irregularly over each of the side surfaces. For example, higher electrical resistance may be provided in edge regions and lower electrical resistance may be provided in a central region of a side surface.

First and second resistance may be identical or symmetric with respect to the center resistance. However, first and second resistance may be different. Depending on an arrangement of the filament arrangement in view of a voltage applied (the first or second contact point being connected to ground or to voltage), there may be slightly different local heating. Different electrical resistance, for example, different filament materials or filament densities in the first and the second side surface may be used to even out differences in heating and thus equilibrate temperature variation over a heater assembly. Consistent heating over an entire heating region of the filament arrangement may thus be supported.

The flat fluid permeable heater assembly according to the invention may also comprise variations of the center resistance or of the first and second resistance, or of the center resistance and the first and second resistance relative to the longitudinal axis.

The heater assembly may, for example, comprise a central longitudinal region extending from the first contact point to the second contact point, wherein an electrical resistance in the central longitudinal region is lower than an electrical resistance outside of the central longitudinal region.

For example, less or smaller filaments may be arranged in edge regions along the filament arrangement than in the central longitudinal region. For example, a mesh density may be higher in the central longitudinal region than in lateral longitudinal regions along the filament arrangement. By this, a power distribution may be concentrated onto a central region of a central surface. Such a specific power distribution may, for example, be realized by a flat filament arrangement wherein in the direction of the longitudinal axis more filaments are arranged in the central longitudinal region than outside the central longitudinal region.

The electrical resistance may be defined and varied by the selection of the material used for the filament arrangement or by the size and arrangement of filaments in the filaments arrangement. Preferably, the electrical resistance is, by a preselected filament material, defined by a ratio of open area to the total area of the filament arrangement.

For example, the fluid permeable heater assembly may comprise an electrically conductive flat filament arrangement, and a first contact point and a second contact point for electrically contacting the flat filament arrangement. A longitudinal axis is defined between the first contact point and the second contact point. In the heater assembly, a center surface Sc is an area of the heater assembly extending between two lines lying perpendicular to the longitudinal axis and crossing the longitudinal axis at two points arranged on the longitudinal axis, one of the two points being situated at a distance from the first contact point equal to 40 percent and the other one of the two points being situated at a distance from the first contact point equal to 60 percent of the distance between the first and the second contact point. A first side surface S1 is an area of the heater assembly extending between two lines lying perpendicular to the longitudinal axis and crossing the longitudinal axis at the first contact point and a point arranged on the longitudinal axis and situated at a distance from the first contact point equal to 20 percent of the distance between the first and the second contact point. A second side surface S2 is an area of the heater assembly between two lines lying perpendicular to the longitudinal axis and crossing the longitudinal axis at the second contact point and a point arranged on the longitudinal axis and situated at a distance from the first contact point equal to 80 percent of the distance between the first and the second contact point.

The center surface Sc comprises a plurality of openings defining an open area ScOA, the first side surface S1 comprises a plurality of openings defining an open area S10A, and the second sidesurface S2 comprises a plurality of openings defining an open area S2OA. A ratio of the open area of the center surface to the open area of the first side surface ScOA/S1OA is between 1.1 and 30, and a ratio of the open area of the center surface to the open area of the second side surface ScOA/S2OA is between 1.1 and 30. Preferably, the ratio of the open area of the center surface to the first side surface or to the second side surface, ScOA/S1OA, or ScOA/S2OA are between 2 and 28, for example between 2 and 15 or between 15 and 28.

The open area of the center surface ScOA may be between about 40 percent and about 90 percent of the total area of the center surface. Preferably, the open area in the center surface is between about 50 percent and about 80 percent, more preferably between about 50 and about 70 percent.

A heater assembly may have a constant width along the length of the longitudinal axis with respect to the filament arrangement.

A heater assembly may have a varying width along the length of the longitudinal axis. In these cases, for the purpose of calculating the open areas, the heater assembly is considered to be the rectangular area between two lines parallel to the longitudinal axis passing through points of the filament arrangement which are the most distant to the longitudinal axis. By this, the absence of filament arrangement in narrower parts of the heater assembly is counted as open area.

Most of the heating may happen in a central surface of the heater assembly between the two contact points. Little heating may happen in the side surfaces.

The open area of the center surface is formed by a plurality of openings, which preferably has a size and distribution optimized for a fluid to be vaporized to penetrate into the openings and allow an as direct and efficient heating of the fluid.

An open area of each side surfaces is smaller than the open area of the center surface. However, preferably, the open area of the first side surface is not larger than about 10 percent of the total area of the first side surface and the open area of the second side surface is also not larger than about 10 percent of the total area of the second side surface. The open area of the side surfaces may each be in a range between about 5 and about 35 percent, for example between about 5 and about 20 percent or between about 5 and about 15 percent of the total area of a side surface.

Small or little open area in side surfaces may enhance an electrical contact in these side surfaces compared to, for example, meshes having low densities, for example, like meshes preferred for center surfaces of a filament arrangement.

In addition, a plurality of openings in side surfaces may limit leakage of liquid out of the heater assembly. Typically, liquid is supplied from a liquid storage reservoir, for example a tank system or cartridge to the heater assembly. The liquid penetrates into the plurality of openings in the center surface where the liquid may be heated and vaporized.

Liquid tends, for example via capillary forces, to pass between a heater substrate and contact portions radially outwardly of the heater. This effect may be substantial when using foils as contact portions as in prior art filament arrangements.

By providing a plurality of openings in the side surfaces, the liquid will enter into the openings and thus be kept in the side surfaces.

Yet further, an overmoulding of contact portions is facilitated. Overmoulding is typically used for stability purposes of contact portions, for example when using thin contact foils or loose meshes. Side surfaces may be overmoulded, for example with a heat resistive polymer. Overmoulding may prevent displacement of individual filaments, or an unravelling of filament edges. With an overmoulding of side surfaces or entire contact portions stability of the side surfaces may be enhanced. This may facilitate mounting of the filament arrangements when assembling a heater assembly. It may also facilitate keeping a form and shape of the filament arrangement. Reproducibility and reliability of heaters using a filament arrangement may thus be improved.

An overmoulding material may be any material suitable for use in a fluid permeable heater according to the invention. An overmoulding material may for example be a material that is able to tolerate high temperatures (in excess of 300 degree Celsius), for example polyimide or thermoplastics such as for example polyetheretherketone (PEEK).

In the filament arrangement, the overmoulding material may penetrate into the openings in the first and second side surfaces. The openings may, for example, form microchannels in the filament arrangement. Thus, a connection between the material of the filament arrangement and the overmoulding material may be enhanced. The low value of open area, in particular small sized openings, may additionally support that the overmoulding material is kept in the side surfaces and does not flow through.

With the filament arrangement provided with a plurality of openings, leakage may be prevented or reduced also with overmoulded side surfaces. Due to a surface of the overmoulded side surface not being flat, surface irregularities may serve as liquid retention.

A ratio of open areas or a value of an open area in the center surface of a filament arrangement or the number, sizes and arrangement of the openings of the plurality of openings in the center surface may, for example, be chosen according to a liquid to be evaporated (viscosity, evaporation temperature, amount of evaporated substance etc.).

A ratio of open areas or a value of an open area in the first and second side surface of a filament arrangement may, for example, be selected according to a heating regime through the filament arrangement or according to the way of contacting the filament arrangement to a heater substrate or contacting the heater assembly. The value of an open area in the two side surfaces may also be selected, for example, according to an overmoulding material used (flow speed, temperature during overmoulding etc.).

The plurality of openings in the side surfaces may be arranged homogenously and regularly over each of the two side surfaces.

The plurality of openings in the side surfaces may be arranged irregularly over each of the side surfaces. For example, more or larger openings may be provided in edge regions and smaller or fewer openings may be provided in a central region of the side surface.

Amount and distribution of openings in the two side surfaces may be identical or symmetric with respect to the center surface. However, amount and distribution of openings in the two side surfaces may be different in the two side surfaces, for example, to even out differences in heating due to a specific power application to the filament arrangement.

A transition surface arranged between a side surface and the center surface may comprise an open area gradient ranging from an open area of a side surface to an open area of the center surface.

The flat filament arrangement may, for example, be a perforated sheet. The center surface of the perforated sheet may comprise a plurality of heater filaments separated or distanced from each other by a plurality of openings. The side surfaces of the perforated sheet may each comprise a plurality of openings.

The openings may, for example, be manufactured by chemical etching or laser treatment.

The flat filament arrangement may, for example, be a mesh arrangement, wherein a mesh of the center surface and meshes of the first and second side surface each comprise a mesh density. The mesh density in the center surface is lower than the mesh density in each of the first and second side surface. Thus, the electrical resistance is lower in the two side surfaces than in the center surface. Interstices between filaments of the meshes define the open area of the center surface and the open areas of each of the first and second side surface.

Mesh arrangements may be manufactured by weaving applying different weaving modes to manufacture the different surface of the mesh. By this, a single strip or a continuous band of mesh may be manufactured having different density meshes in the side surfaces and the center surface. A continuously produced band of mesh may be cut to appropriately sized strips of mesh.

The filament arrangement may be manufactured at low cost, in a reliable and repeatable manner. The filament arrangement may be manufactured in one manufacturing step, not requiring assembly of individual filament arrangement parts.

In a mesh arrangement, a mesh density gradient corresponding to an electrical resistance gradient may be located between the first side surface and the center surface and between the center surface and the second side surface. These mesh gradients may represent transition surfaces between center surface and side surfaces.

The mesh of the center surface may comprise a weft aperture having a same size than a warp aperture of the mesh of the center surface. By this a mesh having regular square-shaped openings in the center surface may be manufactured. The meshes of the first and the second side surface may comprise a weft aperture larger than zero and no warp aperture. By this, very small, regularly arranged openings in the meshes of the two side surfaces may be manufactured.

Preferably, in weaving direction of the filament arrangement a same number of (warp) filaments are arranged next to each other along the entire length of the filament arrangement. In these embodiments, continuing warp filaments extend preferably at least from a first side surface to the second side surface, and more preferably along the entire length of the filament arrangement. By this method, mesh arrangements may be manufactured, wherein a warp aperture in the two side surfaces is equal to the warp aperture of the center surface.

Preferably, the filament arrangement is a mesh arrangement.

For the filaments of the filament arrangement any electrically conductive material suitable for manufacturing a filament arrangement and for being heated may be used.

Preferred materials for the filament arrangement are metals, including metal alloys, and carbon fibers. Carbon fibers may be added to metals or other carrier material to vary the resistance of the filaments.

Filament diameters may be in a range between about 8 micrometer and about 50 micrometer, preferably between about 10 micrometer and about 30 micrometer, more preferably between 12 micrometer and about 20 micrometer, for example about 16 micrometer.

Side surfaces made of mesh may be compressed. By this, electrical contact between individual filaments of the mesh and thus contact with the filament arrangement may be improved.

Sizes of openings in the center surface may, for example, have a length and width or diameter between about 25 micrometer and about 75 micrometer, for example a length and width or diameter between about 60 and about 80 micrometer.

Sizes of openings in the side surfaces may, for example have length and width between about 0.5 micrometer and about 75 micrometer. Preferably, sizes of openings in side surfaces have, for example, a width up to about 75 micrometer, when a length decreases versus about 0.5 micrometer. Preferably, sizes of openings in side surfaces have diameters between about 5 micrometer and about 50 micrometer or corresponding opening areas.

The center surface of the flat filament arrangement may have a size in a range, for example, between about 5 mm² and about 35 mm², for example in a range between about 10 mm² and about 30 mm², for example about 25 mm². Preferably, a center surface has a rectangular, preferably substantially square form, for example about 5x5 mm². Heat dissipation may be kept low in surfaces having about a same length and width.

A side surface may have a size, for example in a range between about 3 mm² to about 15 mm², for example in a range between about 5 mm² to about 10 mm², for example about 5 mm² or about 10 mm².

Depending on the position of contacts or contact points on the filament, the distance between the contact points may be equal to a total length of the filament arrangement. Typically, the distance between two contact points is shorter than the total length of the filament arrangement. Preferably, the specification of the remaining longitudinal ends of the filament arrangement longitudinally extending beyond the contact points is equal or similar to the specifications of the side surfaces and as described herein. In particular, the longitudinal ends of the mesh filament preferably comprise a resistance and open area as the side surfaces.

Preferably, side surfaces have the form of strips, for example a rectangular strip of about 5x(1-2) mm².

The sizes of contact portions or side surfaces, respectively, may be adapted to provide good contact with connectors used to connect the heater assembly to a power supply, for example a contact with pogo pins.

A number of openings of the plurality of openings in the center surface may, for example, be in a range between about 5 and about 100 openings per mm², preferably between about 15 and about 70 openings per mm², for example about 40 openings per mm².

A number of openings of the plurality of openings in a side surface may, for example, be in a range between about 20 and about 400 openings per mm², preferably between about 50 and 350 openings per mm², for example about 300 to about 350 openings per mm².

A filament arrangement may be pretreated. Pretreatment may be a chemical or physical pretreatment, for example, changing the surface characteristic of the filament surface. For example, a filament surface may be treated to enhance wettability of the filament, preferably in a center surface or heating region only. Increased wettability has been found particularly favorable for liquids typically used in electronic vaporization devices, so called e-liquids. E-liquids typically comprise an aerosol-former such as glycerol or propylene glycol. The liquids may additionally comprise flavourants or nicotine.

The aerosol-forming liquids evaporated by a heated filament arrangement according to the invention comprises at least one aerosol former and a liquid additive.

The aerosol-forming liquid may comprise water.

The liquid additive may be any one or a combination of a liquid flavour or liquid stimulating substance. Liquid flavour may for example comprise tobacco flavour, tobacco extract, fruit flavour or coffee flavour. The liquid additive may, for example, be a sweet liquid such as for example vanilla, caramel and cocoa, a herbal liquid, a spicy liquid, or a stimulating liquid containing, for example, caffeine, taurine, nicotine or other stimulating agents known for use in the food industry.

Advantageously, the fluid permeable heater assembly comprises a substrate comprising an opening through the substrate. The electrically conductive flat filament arrangement extends over the opening in the substrate. The heater assembly further comprises fastener attaching the flat filament arrangement to the substrate.

The fastener may itself be electrically conductive and may serve as electrical contact for providing heating current through the filament arrangement.

The fastener may be chemical or mechanical fastener. The filament arrangement may, for example be attached to the substrate by bonding or gluing.

Preferably, the fastener are mechanical fastener such as clamps, screws or form-locking fastener. Clamps and flat heater assemblies using clamps to clamp a filament arrangement to a heater substrate have been described in detail in the international patent publication WO2015/117701. Reference is herewith made to this international patent publication WO2015/117701 and its content relating to the heater assembly and clamps used and described therein is incorporated herewith.

The fastener may be one or a combination of the aforementioned fastener.

Preferably, the heater assembly is a flat heater assembly, preferably, a resistively heatable fluid permeable flat heater assembly.

According to the invention, there is also provided an electrically operated aerosol-generating system. The system comprises an aerosol-generating device and a cartridge comprising a liquid aerosol-forming substrate. The system further comprises a fluid permeable heater assembly according to the invention and as described herein for heating liquid aerosol-forming substrate. The cartridge comprises a housing having an opening, with the heater assembly extending across the opening of the housing of the cartridge. The aerosol-generating device comprises a main body defining a cavity for receiving the cartridge, an electrical power source, and electrical contacts for connecting the electrical power source to the heater assembly, that is, to the first and second contact points of the heater assembly, for heating the filament arrangement.

Preferably, the cartridge comprises a liquid comprising at least an aerosol-former and a liquid additive.

Features and advantages of the aerosol-generating system have been described relating to the heater assembly according to the invention.

The invention is further described with regard to embodiments, which are illustrated by means of the following drawings, wherein:
- Fig. 1: is a schematic illustration of resistance distribution over a heater assembly;
- Fig. 2: is a schematic illustration of mesh arrangement;
- Fig. 2a: is a schematic illustration of a resistance distribution of the mesh arrangement of Fig.2;
- Fig. 3: is an exploded view of a heater assembly with mesh arrangement;
- Fig. 4: shows the assembled heater assembly of Fig. 3;
- Fig. 5: shows a heater substrate with mesh arrangement;
- Fig. 6: is an enlarged view of Fig.5;
- Fig. 7: shows enlarged views of transition and contact regions of a mesh arrangement;
- Fig. 8: shows a tin-plated contact region of a mesh heater;
- Fig. 9: is a schematic illustration of another embodiment of a mesh arrangement;

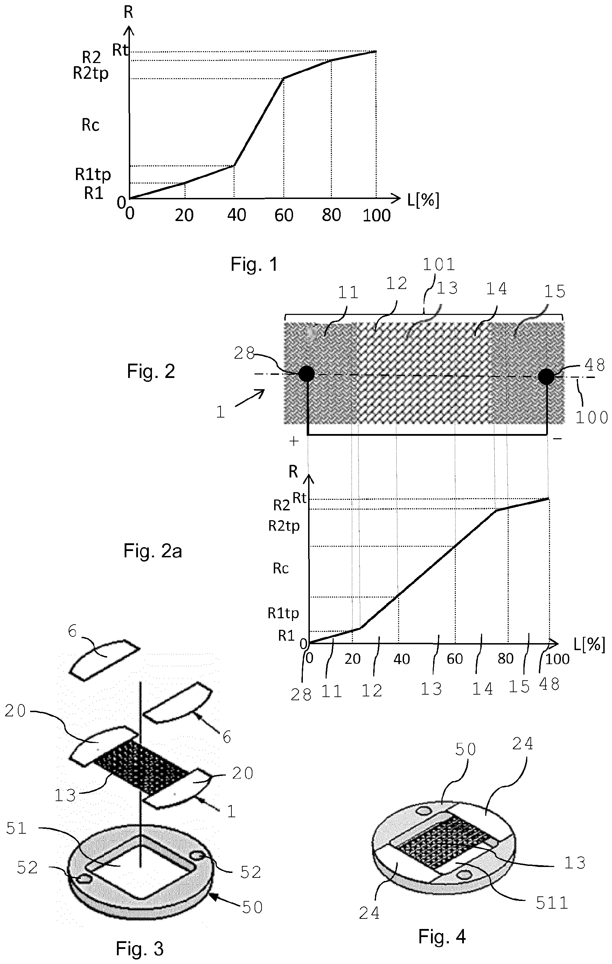

In **Fig. 1** a schematic illustration of an example of a resistance distribution along the longitudinal axis 100 of a heater assembly between a first contact point at position 0% and a second contact point at position 100% is shown. The vertical axis indicates the resistance (R) of the heater assembly up to a total resistance Rt of the heater assembly. The horizontal axis (L[%])) indicates the position on the longitudinal axis from the first contact point to the second contact point.

In the example of Fig. 1, the heater assembly comprises a first resistance R1 which is present over 20 percent along the longitudinal axis starting at the first contact point at 0 into the direction of the second contact point. A first transition resistance R1tp is present between 20 percent and 40 percent along the longitudinal axis. A center resistance Rc is present between 40 percent and 60 percent along the longitudinal axis and after the first contact point. A second transition resistance R2tp is present from a point between 60 percent and 80 percent along the longitudinal axis after the first contact point. A second resistance is present from 80 percent to 100 percent, that is, over the last 20 percent of the heater assembly along the longitudinal axis between the first and second contact point.

The heater assembly is contacted in the first and the second contact points and a current is allowed to flow through the filament arrangement of the heater assembly.

The first resistance R1 may be up to a maximum of 13 percent of the total resistance Rt and as low as 0.5 percent of the total resistance Rt.

The first and second transition resistances R1tp, R2tp are each not higher than the center resistance in order to prevent extensive heating in a transition surface of a heater assembly. Typically, the first and second transition resistance R1tp, R2tp have a value in between the first resistance R1 and the center resistance Rc or the center resistance Rc and the second resistance R2, respectively. The center resistance Rc is about 50 percent of the total resistance Rt of the heater assembly. Preferably, the center resistance Rc is more than 50 percent of the total resistance Rt. The second resistance may be up to a maximum of 13 percent of the total resistance Rt and as low as 0.5 percent of the total resistance Rt.

The first and second resistance R1, R2, the first and second transition resistance R1tp, R2tp and the center resistance Rc add up to the total resistance Rt of the heater assembly.

In **Fig. 2** a mesh arrangement 1 for a resistively heatable flat fluid permeable heater is shown. The mesh arrangement has a rectangular shape having a length 101 (Lf). The mesh filament may be contacted, for example by a pogo pin, in one spot as indicated by contact points 28,48. Over the contact points 28,48 a voltage is applied.

When arranged in a heater assembly and contacted in contact points 28, 48, areas of the filament arrangement define heater surfaces each extending over 20 percent of the distance between the first contact point 28 and the second contact point 48.

A longitudinal axis 100 is defined between the first and second contact point 28, 48, which longitudinal axis corresponds to a central longitudinal axis of the filament arrangement 1. Along the longitudinal axis 100 the resistance of the heater surface is measured (see Fig. 1).

A first side surface 11 extends from the first contact point 28 over 20 percent of the distance between first and second contact point 28, 48 along the longitudinal axis into the direction of the second contact point 48.

A first transition surface 12 extends between 20 percent and 40 percent of the distance between first and second contact point 28, 48 along the longitudinal axis.

A center surface 13 extends between 40 percent and 60 percent of the distance between first and second contact point 28, 48 along the longitudinal axis.

A second transition surface 14 extends between 60 percent and 80 percent of the distance between first and second contact point 28, 48 along the longitudinal axis.

A second side surface 15 extends between 80 percent and 100 percent of the distance between first and second contact point 28, 48 along the longitudinal axis counted from the first contact point 28 into the direction of the second contact point 48.

The center surface 13 comprises a low mesh density over its entire surface.

The first and second side surfaces 11, 15 comprise a high mesh density over their entire surface.

The first and second transition surfaces 12, 14 comprise parts with a high mesh density and parts with a low mesh density.

The center surface 13 is designed to be the main heating region of the mesh arrangement.

In Fig. 2 all heater surfaces have a rectangular shape and the two side surfaces 11, 15 have a same size.

The meshes of the first and the second side surfaces 11, 15 have a higher density than the mesh of the central surface 13. Preferably, the densities of the meshes of the side surfaces are identical. The mesh densities of the side surfaces may also be different, for example to compensate for a different size of the mesh filaments in these regions or for example to even out heating differences due to a flow direction of a current flowing through the mesh arrangement.

The meshes of the side surfaces 11, 15 have an open area formed by the sum of the interstices between the filaments of the meshes of preferably less than 20 percent of the total area of each of the first and second side surfaces. Thus, in the first and second side surfaces 11, 15 an open area is each preferably about maximal 1 mm², with a total size of each of the first and second side surfaces of about 4-5 mm².

The current flowing between the contact points 28, 48 causes resistive heating of the mesh filament in the center surface 13 and in the transition surfaces 12, 14 according to their higher resistance.

In **Fig. 2a** a schematic resistance distribution of the mesh arrangement of Fig. 2 is shown.

In Fig. 2a the resistance distribution is indicated along the longitudinal axis 100 between the first and the second contact points 28, 48.

Typically, the contact points 28, 48 are not arranged at the extreme ends of the filament arrangement. Thus, not the entire length 101 of the filament arrangement contributes to the resistance of a heater assembly comprising the filament arrangement.

The mesh arrangement of Fig. 2 does not have any transition portions with, for example, a mesh density gradient. Thus, the first transition resistance R1tp is at first equal to the first resistance R1 in the side surface 11 and then equal to the center resistance Rc of the center surface 13. Accordingly, the second transition resistance R2tp is at first equal to the center resistance Rc of the center surface 13 and then equal to the second resistance R2 of the second side surface 15 when seen in a direction from the first contact point 28 to the second contact point 48 along the longitudinal axis 100. Thus, a heating region of the mesh arrangement of Fig. 2 comprising a low mesh density and a high resistance extends over about 50 percent of the filament arrangement. The two side surfaces 12, 15 comprising a low mesh density and a low first and second resistance R1, R2 each extend over 20 percent of distance between the two contact points 28, 48.

**Fig. 3** and **Fig. 4** schematically show an example of a setup of a flat, fluid-permeable heater assembly with a mesh arrangement. In the exploded view of the heater in Fig. 3 an electrically insulating substrate 50, a heater element and filament arrangement in the form of a mesh arrangement 1 and two metal sheets 6 are shown. The metal sheets may, for example, be sheets of tin, to alter electrical contact of connectors, for example contact pins, with the longitudinal ends 20 of the mesh arrangement 1.

The substrate 50 has the form of a circular disc and comprises a centrally arranged opening 51. The substrate comprises two bore holes 52 arranged diagonally opposite each other in the substrate. The bore holes 52 may serve for positioning and mounting the heater assembly for example in an aerosol-generating device.

The mesh arrangement 1 comprises a central surface 13 and in the embodiment shown in Figs. 3 and 4 two PEEK overmoulded longitudinal ends 20. The mesh arrangement is arranged over the square-formed centrally arranged opening 51 and over the substrate 50. The entire central surface 13 of the mesh arrangement including those portions of the transition surfaces comprising a low mesh density come to lie over the opening 51. The two longitudinal ends 20, in particular those portions of the longitudinal ends overmoulded with PEEK and tin-plated (covered with the metal sheets 6) come to lie on the substrate 50.

The width of the mesh of the central surface13 is smaller than the width of the opening 51 such that on both lateral sides of the central surface 13 an open portion 511 of the opening 51 is formed. The open portions 511 are not covered by mesh. The tin-plated dense mesh of the longitudinal ends forms a more plane contact area 24 than the mesh itself. The contact area 24 is arranged parallel to the top surface of the substrate 50 of the heater assembly. The contact areas 24 are for contacting the heater assembly by an electrical connector from for example a battery.

Fig. 4 shows the heater assembly of Fig. 3 in an assembled state. The mesh arrangement 1 may be attached to the substrate 50 by mechanical means or for example by adhesive.

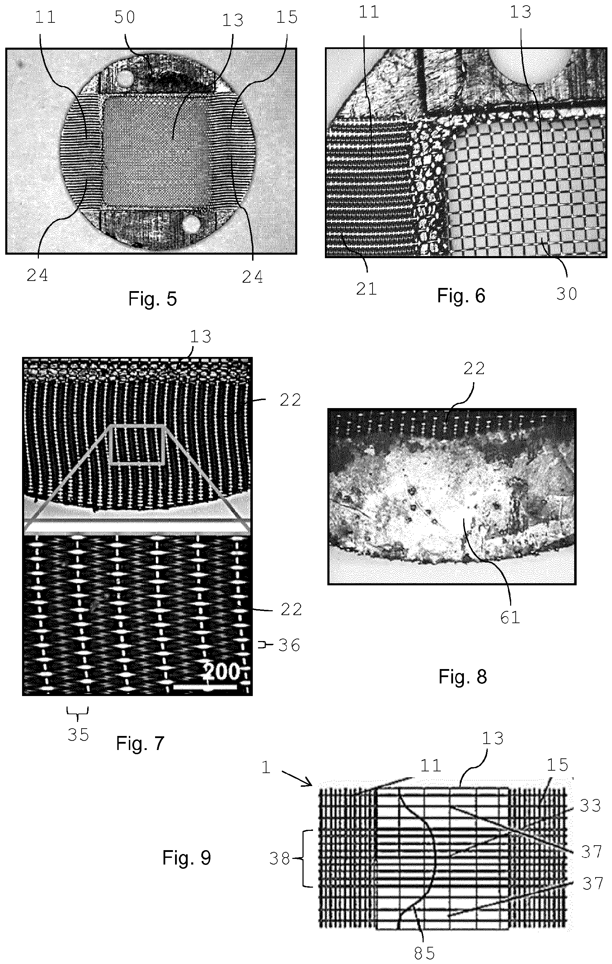

**Fig. 5** shows a heater substrate 50 with a mesh arrangement 1 attached thereto. The mesh arrangement is a rectangular strip of mesh with a high density mesh in contact areas 24 of the heater assembly and a low density mesh in between defining the heating region of the heater assembly.

This may better be seen in **Fig. 6****,** which is an enlarged view of a detail of Fig. 5. The low density mesh of the center surface 13 of the mesh arrangement has rectangular interstices 30 in a micrometer range, for example 70 micrometer. With a wire diameter of the filaments of 16 micrometer, the open area of the center surface covers about 75 percent of the total area of the center surface.

The high density mesh of the side surface 11 of the mesh arrangement has smaller interstices 21 of about 0.1 micrometer x 5 micrometer. With a filament diameter of 16 micrometer, the open area of the side surfaces covers about 3 percent of the total area of each of the side surfaces.

The mesh arrangement has been produced in one piece by different weaving modes.

The amount of filaments in a weaving direction is identical over the entire filament arrangement. The weaving direction corresponds to the warp direction of the filament arrangement, which warp direction corresponds to the main current flow direction in the mesh arrangement. However, the weaving density of the filaments in weft direction (perpendicular to the warp direction) is enhanced in the side surface 11. A distance between filaments in the weft direction may be reduced to zero in the side surfaces 11, 15.

Depending on the production mode, a transition in mesh density may be provided between center surface 13 and side surface 11, for example a density gradient in mesh density. Preferably, such density gradient smoothly changes from the low density of the mesh of the center surface to the higher density of the mesh of the side surface and vice versa.

In **Fig. 7** the higher density mesh in the side surface 22 has been compressed to improve electrical contact between the individual filaments of the mesh. A filament to filament distance between warp filaments 35 is about 25 micrometer to 75 micrometer, in Fig. 7 about 70 micrometer. The filament to filament distance of weft filaments 36 is zero. The open area in the side surfaces is generated by the manufacturing of the filament arrangement through weaving.

To improve electrical contact of the longitudinal ends of the mesh arrangement, an outermost part of the compressed ends, at least partly including the side surface 22, is tin-plated 61 as may be seen in **Fig. 8****.**

**Fig. 9** shows a mesh arrangement 1 having a first side surface 13, an intermediate central surface 13 and an opposite second side surface 15. The mesh density in the two side surfaces 11,15 is higher than the mesh density in the center surface 13. The mesh arrangement 1 comprises a longitudinal central portion 38 arranged along a longitudinal central axis 100 of the mesh arrangement 1. The mesh density in this longitudinal central portion 38 is higher than outside in lateral side regions 37 of the mesh arrangement. The longitudinal central portion 38 has a width of about 50% to 60% of the total width of the mesh arrangement 1.

The higher mesh density in a central region 33 of the central surface leads to a high power density in this region and concentrates the main heating zone to this central region 33 of the center surface 13. Due to the different mesh densities in the different regions of the mesh arrangement, the highest power density is in the middle or central region 33 of the center surface 13. The lower density areas in the lateral regions 37 in the central surface 13 have comparably high resistance. The power density curve over the width of the central surface 13 is shown with line 85.

The side surfaces 11, 15 form part of high density mesh contact pads with comparably low resistance. Preferably, the electrical contacts are arranged on the longitudinal axis in the side surfaces 11, 15, where an electrical resistance is lowest in the side surfaces.

The examples shown in the figures typically have symmetric side surfaces with a same size and a same mesh density or density distribution. Such embodiments simplify a manufacturing and symmetric arrangement of a heater assembly. However, asymmetric mesh arrangement and mesh gradients may easily be provided to achieve a desired power distribution regime in the mesh filament.

As becomes obvious from the resistance distribution, side surfaces of filament arrangements may for example be smaller or larger, have more and smaller or less and larger openings, be smaller and have higher mesh density or be larger and have lower mesh density, all in order to achieve a same or a specific resistance regime in the surfaces of the heater assembly. Such variations allow much flexibility in the application of the heater assembly. For example, it enables to adapt the heater assembly to various liquids to be aerosolized, for example more or less viscous fluids.

The filament arrangement may easily be adapted to differently sized heaters or to aerosol-generating devices having more or less power available for heating a heater assembly.

## Claims

1. Fluid permeable heater assembly for aerosol-generating systems, the fluid permeable heater assembly comprises an electrically conductive flat filament arrangement, and
a first contact point (28) and a second contact point (48) for electrically contacting the flat filament arrangement, wherein a longitudinal axis is defined between the first contact point (28) and the second contact point (48),
wherein a center resistance Rc is the electrical resistance between two points situated on the longitudinal axis, one of the two points being situated at a distance from the first contact point (28) equal to 40 percent and the other one of the two points being situated at a distance from the first contact point (28) equal to 60 percent of the distance between the first and the second contact point (28, 48);
wherein a first resistance R1 is an electrical resistance between the first contact point (28) and a point situated on the longitudinal axis at a distance from the first contact point (28) equal to 20 percent of the distance between the first and the second contact point (28, 48);
wherein a second resistance R2 is an electrical resistance between the second contact point (48) and a point situated on the longitudinal axis at a distance from the first contact point (28) equal to 80 percent of the distance between the first and the second contact point (48);
and wherein a ratio of the center resistance to the first resistance Rc/R1 is between 2 and 400, and wherein a ratio of the center resistance to the second resistance Rc/R2 is between 2 and 400.

2. Heater assembly according to claim 1, comprising a total resistance Rt corresponding to the electrical resistance between the first contact point (28) and the second contact point (48),
wherein a ratio of the center resistance to the total resistance Rc/Rt corresponds to at least 0.5;
wherein a ratio of the first resistance to the total resistance R1/Rt is between 0.005 and 0.125, and wherein a ratio of the second resistance to the total resistance R2/Rt is between 0.005 and 0.125.

3. Heater assembly according to any one of the preceding claims, further comprising:
a first transition resistance R1tp corresponding to the electrical resistance between two points situated on the longitudinal axis, one of the two points being situated at a distance from the first contact point (28) equal to 20 percent and the other one of the two points being situated at a distance from the first contact point (28) equal to 40 percent of the distance between the first and the second contact point (28, 48); and
a second transition resistance R2tp corresponding to the electrical resistance between two points situated on the longitudinal axis, one of the two points being situated at a distance from the first contact point (28) equal to 60 percent and the other one of the two points being situated at a distance from the first contact point (28) equal to 80 percent of the distance between the first and the second contact point (28, 48);
wherein a ratio of the first transition resistance to the first resistance R1tp/R1 is between 1.1 and 400,
wherein a ratio of the second transition resistance to the second resistance R2tp/R2 is between 1.1 and 400,
and wherein a ratio of the center resistance to the first transition resistance Rc/R1tp is between 1.1 and 400,
and wherein a ratio of the center resistance to the second transition resistance Rc/R2tp is between 1.1 and 400.

4. Heater assembly according to any one of the preceding claims, wherein a total resistance Rt corresponding to the electrical resistance between the first contact point (28) and the second contact point (48) is between 0.5 Ohm and 4 Ohm, wherein the center resistance Rc is higher than 0.5 Ohm, wherein the first resistance R1 and the second resistance R2 are each lower than 100 mOhm.

5. Heater assembly according to any one of the preceding claims, comprising a central longitudinal region extending from the first contact point (28) to the second contact point (48), wherein an electrical resistance in the central longitudinal region is lower than an electrical resistance outside of the central longitudinal region.

6. Heater assembly according to any one of the preceding claims, wherein the electrically conductive flat filament arrangement is a perforated sheet, with a center surface of the perforated sheet comprising a plurality of heater filaments and with a first and second side surface of the perforated sheet comprising a plurality of openings, the first and second side surfaces being arranged on opposite sides of the center surface, the first side surface comprising the first contact point (28) and the second side surface comprising the second contact point (48).

7. Heater assembly according to any one of claims 1 to 5, wherein the electrically conductive flat filament arrangement is a mesh arrangement (1) comprising a center surface (13) and a first and a second side surface (11, 15), wherein a mesh of a center surface (13) and meshes of first and second side surfaces (11, 15) each comprise a mesh density, wherein the mesh density in the center surface (13) is lower than the mesh density in each of the first and second side surfaces (11, 15), wherein the first and second side surfaces are arranged on opposite sides of the center surface (13), the first side surface (11) comprising the first contact point (28) and the second side surface (15) comprising the second contact point (48).

8. Heater assembly according to claim 7, wherein a mesh density gradient is located between the first side surface (11) and the center surface (13) and between the center surface (13) and the second side surface (15).

9. Heater assembly according to any one of claims 7 to 8, wherein the meshes of the first and the second side surface (11, 15) comprise a weft aperture larger than zero and no warp aperture.

10. Heater assembly according to any one of claims 7 to 9, wherein in weaving direction of the filament arrangement a same number of filaments are arranged next to each other in the center surface and in the first and the second side surfaces (11, 15).

11. Heater assembly according to any one of claims 7 to 10, wherein in weaving direction of the filament arrangement more filaments are arranged in a central longitudinal region than outside the central longitudinal region.

12. Heater assembly according to any one of the preceding claims, further comprising:
a substrate comprising an opening through the substrate, the electrically conductive flat filament arrangement extending over the opening in the substrate; and a fastener attaching the flat filament arrangement to the substrate.

13. Heater assembly according to claim 12, wherein the fastener is electrically conductive and serves as electrical contact for providing heating current through the filament arrangement.

14. Heater assembly according to any one of claims 12 to 13, wherein the fastener is a mechanical fastener such as clamps, screws or form-locking fastener.

15. Electrically operated aerosol-generating system comprising:
an aerosol-generating device and a cartridge comprising a liquid aerosol-forming substrate;
a fluid permeable heater assembly according to any one of claims 1 to 14 for heating liquid aerosol-forming substrate,
wherein the cartridge comprises a housing having an opening, with the heater assembly extending across the opening of the housing of the cartridge,
and wherein the aerosol-generating device comprises a main body defining a cavity for receiving the cartridge, an electrical power source, and electrical contacts for connecting the electrical power source to the heater assembly.

## Patentansprüche

1. Fluiddurchlässige Heizvorrichtungsbaugruppe für Aerosolerzeugungssysteme, wobei die fluiddurchlässige Heizvorrichtungsbaugruppe eine elektrisch leitfähige, flache Fadenanordnung aufweist, und
eine erste Kontaktstelle (28) und eine zweite Kontaktstelle (48) für den elektrischen Kontakt der flachen Fadenanordnung, wobei eine Längsachse zwischen der ersten Kontaktstelle (28) und der zweiten Kontaktstelle (48) definiert ist,
wobei der Mittelwiderstand Rc der elektrische Widerstand zwischen zwei auf der Längsachse liegenden Punkten ist, wobei der eine der beiden Punkte in einem Abstand von der ersten Kontaktstelle (28) gleich 40 % liegt und der andere der beiden Punkte in einem Abstand von der ersten Kontaktstelle (28) gleich 60 % des Abstands zwischen der ersten und der zweiten Kontaktstelle (28, 48) liegt;
wobei ein erster Widerstand R1 ein elektrischer Widerstand zwischen der ersten Kontaktstelle (28) und einem auf der Längsachse liegenden Punkt in einem Abstand von der ersten Kontaktstelle (28) gleich 20 % des Abstands zwischen der ersten und der zweiten Kontaktstelle (28, 48) ist;
wobei ein zweiter Widerstand R2 ein elektrischer Widerstand zwischen der zweiten Kontaktstelle (48) und einem auf der Längsachse liegenden Punkt in einem Abstand von der ersten Kontaktstelle (28) gleich 80 % des Abstands zwischen der ersten und der zweiten Kontaktstelle (48) ist;
wobei ein Verhältnis des Mittelwiderstandes zu dem ersten Widerstand Rc/R1 zwischen 2 und 400 ist, und wobei ein Verhältnis des Mittelwiderstands zu dem zweiten Widerstand Rc/R2 zwischen 2 und 400 ist.

2. Heizvorrichtungsbaugruppe nach Anspruch 1, die einen Gesamtwiderstand Rt entsprechend dem elektrischen Widerstand zwischen der ersten Kontaktstelle (28) und der zweiten Kontaktstelle (48) aufweist,
wobei ein Verhältnis des Mittelwiderstands zum Gesamtwiderstand Rc/Rt zumindest 0,5 entspricht;
wobei ein Verhältnis des ersten Widerstands zum Gesamtwiderstand R1/Rt zwischen 0,005 und 0,125 ist, und wobei ein Verhältnis des zweiten Widerstands zum Gesamtwiderstand R2/Rt zwischen 0,005 und 0,125 ist.

3. Heizvorrichtungsbaugruppe nach einem der vorhergehenden Ansprüche, ferner aufweisend:
einen ersten Übergangswiderstand R1tp, der dem elektrischen Widerstand zwischen zwei auf der Längsachse liegenden Punkten entspricht, wobei einer der beiden Punkte in einem Abstand von der ersten Kontaktstelle (28) gleich 20 % liegt und der andere der beiden Punkte in einem Abstand von der ersten Kontaktstelle (28) gleich 40 % des Abstands zwischen der ersten und der zweiten Kontaktstelle (28, 48) liegt; und
ein zweiter Übergangswiderstand R2tp, der dem elektrischen Widerstand zwischen zwei auf der Längsachse liegenden Punkten entspricht, wobei einer der beiden Punkte in einem Abstand von der ersten Kontaktstelle (28) gleich 60 % und
der andere der beiden Punkte in einem Abstand von der ersten Kontaktstelle (28) gleich 80 % des Abstands zwischen der ersten und der zweiten Kontaktstelle (28, 48) liegt;
wobei ein Verhältnis des ersten Übergangswiderstands zum ersten Widerstand R1tp/R1 zwischen 1,1 und 400 ist,
wobei ein Verhältnis des zweiten Übergangswiderstands zu dem zweiten Widerstand R2tp/R2 zwischen 1,1 und 400 ist,
und wobei ein Verhältnis des Mittelwiderstands zu dem ersten Übergangswiderstand Rc/R1tp zwischen 1,1 und 400 ist,
und wobei ein Verhältnis des Mittelwiderstands zu dem zweiten Übergangswiderstand Rc/R2tp zwischen 1,1 und 400 ist.

4. Heizvorrichtungsbaugruppe nach einem der vorhergehenden Ansprüche, wobei ein Gesamtwiderstand Rt, der dem elektrischen Widerstand zwischen der ersten Kontaktstelle (28) und der zweiten Kontaktstelle (48) entspricht, zwischen 0,5 Ohm und 4 Ohm ist, wobei der Mittelwiderstand Rc höher als 0,5 Ohm ist, wobei der erste Widerstand R1 und der zweite Widerstand R2 jeweils niedriger als 100 mOhm sind.

5. Heizvorrichtungsbaugruppe nach einem der vorhergehenden Ansprüche, die einen mittleren Längsbereich umfasst, der sich von der ersten Kontaktstelle (28) zu der zweiten Kontaktstelle (48) erstreckt, wobei ein elektrischer Widerstand in dem mittleren Längsbereich niedriger ist als ein elektrischer Widerstand außerhalb des mittleren Längsbereichs.

6. Heizvorrichtungsbaugruppe nach einem der vorhergehenden Ansprüche, wobei die elektrisch leitende, flache Fadenanordnung ein perforiertes Blatt ist, wobei eine mittlere Oberfläche des perforierten Blattes eine Vielzahl von Heizvorrichtungsfäden aufweist und wobei eine erste und zweite Seitenfläche des perforierten Blatts eine Vielzahl von Öffnungen aufweisen, wobei die erste und zweite Seitenfläche auf gegenüberliegenden Seiten der mittleren Oberfläche angeordnet sind, wobei die erste Seitenfläche die erste Kontaktstelle (28) aufweist und die zweite Seitenfläche die zweite Kontaktstelle (48) aufweist.

7. Heizvorrichtungsbaugruppe nach einem der Ansprüche 1 bis 5, wobei die elektrisch leitende, flache Fadenanordnung eine Netzanordnung (1) ist, die eine mittlere Oberfläche (13) und eine erste und eine zweite Seitenfläche (11, 15) aufweist, wobei ein Netz einer mittleren Oberfläche (13) und Netze der ersten und zweiten Seitenflächen (11, 15) jeweils eine Netzdichte umfassen, wobei die Netzdichte in der mittleren Oberfläche (13) kleiner ist als die Netzdichte in jeder der ersten und zweiten Seitenflächen (11, 15), wobei die erste und die zweite Seitenfläche auf gegenüberliegenden Seiten der mittleren Oberfläche (13) angeordnet sind, wobei die erste Seitenfläche (11) eine erste Kontaktstelle (28) aufweist und die zweite Seitenfläche (15) die zweite Kontaktstelle (48) aufweist.

8. Heizvorrichtungsbaugruppe nach Anspruch 7, wobei ein Netzdichtegradient sich zwischen der ersten Seitenfläche (11) und der mittleren Oberfläche (13) und zwischen der mittleren Oberfläche (13) und der zweiten Seitenfläche (15) befindet.

9. Heizvorrichtungsbaugruppe nach einem der Ansprüche 7 bis 8, wobei die Netze der ersten und der zweiten Seitenflächen (11, 15) eine Schuss-Maschenweite größer als Null und keine Ketten-Maschenweite aufweisen.

10. Heizvorrichtungsbaugruppe nach einem der Ansprüche 7 bis 9, wobei in der Webrichtung der Fadenanordnung eine gleiche Anzahl an Fäden nebeneinander in der mittleren Oberfläche und in der ersten und der zweiten Seitenfläche (11, 15) angeordnet sind.

11. Heizvorrichtungsbaugruppe nach einem der Ansprüche 7 bis 10, wobei in der Webrichtung der Fadenanordnung mehr Fäden in einem mittleren Längsbereich angeordnet sind als außerhalb des mittleren Längsbereichs.

12. Heizvorrichtungsbaugruppe nach einem der vorhergehenden Ansprüche, ferner aufweisend:
ein Substrat, das eine Öffnung durch das Substrat aufweist,
wobei sich die elektrisch leitende, flache Fadenanordnung über die Öffnung in dem Substrat erstreckt; und ein Befestigungselement, das die flache Fadenanordnung an dem Substrat befestigt.

13. Heizvorrichtungsbaugruppe nach Anspruch 12, wobei das Befestigungselement elektrisch leitfähig ist und als elektrischer Kontakt dient, um Heizstrom durch die Fadenanordnung bereitzustellen.

14. Heizvorrichtungsbaugruppe nach einem der Ansprüche 12 bis 13, wobei das Befestigungselement ein mechanisches Befestigungselement wie z. B. Klammern, Schrauben oder ein formschlüssiges Befestigungsmittel ist.

15. Elektrisch betriebenes Aerosolerzeugungssystem aufweisend:
eine Aerosolerzeugungsvorrichtung und Patrone, die ein flüssiges aerosolbildendes Substrat aufweist;
eine fluiddurchlässige Heizvorrichtungsbaugruppe nach einem der Ansprüche 1 bis 14 zum Erwärmen eines flüssigen aerosolbildenden Substrats,
wobei die Patrone ein Gehäuse mit einer Öffnung aufweist,
wobei sich die Heizvorrichtungsbaugruppe über die Öffnung des Gehäuses der Patrone erstreckt,
und wobei die Aerosolerzeugungsvorrichtung einen Hauptkörper aufweist, der einen Hohlraum zur Aufnahme der Patrone, eine elektrische Stromquelle und elektrische Kontakte zur Verbindung der elektrischen Stromquelle mit der Heizvorrichtungsbaugruppe definiert.

## Revendications

1. Ensemble de chauffage perméable aux fluides pour les systèmes de génération d'aérosol, l'ensemble de chauffage perméable aux fluides comprend un agencement de filaments plats électriquement conducteurs, et
un premier point de contact (28) et un deuxième point de contact (48) pour le contact électrique avec l'agencement de filaments plats, dans lequel un axe longitudinal est défini entre le premier point de contact (28) et le deuxième point de contact (48),
dans lequel une résistance centrale Rc est la résistance électrique entre deux points situés sur l'axe longitudinal, l'un des deux points étant situé à une distance du premier point de contact (28) égal à 40 pour cent et l'autre des deux points étant situé à une distance du premier point de contact (28) égal à 60 pour cent de la distance entre le premier et le deuxième points de contact (28, 48) ;
dans lequel une première résistance R1 est une résistance électrique entre le premier point de contact (28) et un point situé sur l'axe longitudinal à une distance du premier point de contact (28) égal à 20 pour cent de la distance entre le premier et le deuxième points de contact (28, 48) ; dans lequel une deuxième résistance R2 est une résistance électrique entre le deuxième point de contact (48) et un point situé sur l'axe longitudinal à une distance du premier point de contact (28) égal à 80 pour cent de la distance entre le premier et le deuxième points de contact (48) ;
et dans lequel un rapport de la résistance centrale à la première résistance Rc/R1 est compris entre 2 et 400, et dans lequel un rapport de résistance centrale à la deuxième résistance Rc/R2 est compris entre 2 et 400.

2. Ensemble de chauffage selon la revendication 1, comprenant une résistance totale Rt correspondant à la résistance électrique entre le premier point de contact (28) et le deuxième point de contact (48),
dans lequel un rapport de la résistance centrale à la résistance totale Rc/Rt correspond à au moins 0,5 ;
dans lequel un rapport de la première résistance à la résistance totale R1/Rt est compris entre 0,005 et 0,125, et dans lequel un rapport de la deuxième résistance à la résistance totale R2/Rt est compris entre 0,005 et 0,125.

3. Ensemble de chauffage selon l'une quelconque des revendications précédentes, comprenant en outre :
une première résistance de transition R1tp correspondant à la résistance électrique entre deux points situés sur l'axe longitudinal, l'un des deux points étant situé à une distance du premier point de contact (28) égal à 20 pour cent et l'autre des deux points étant situé à une distance du premier point de contact (28) égal à 40 pour cent de la distance entre le premier et le deuxième points de contact (28, 48) ; et
une deuxième résistance de transition R2tp correspondant à la résistance électrique entre deux points situés sur l'axe longitudinal, l'un des deux points étant situé à une distance du premier point de contact (28) égal à 60 pour cent et l'autre des deux points étant situé à une distance du premier point de contact (28) égal à 80 pour cent de la distance entre le premier et le deuxième points de contact (28, 48) ;
dans lequel un rapport de la première résistance de transition à la première résistance R1tp/R1 est compris entre 1,1 et 400,
dans lequel un rapport de la deuxième résistance de transition à la deuxième résistance R2tp/R2 est compris entre 1,1 et 400,
et dans lequel un rapport de la résistance centrale à la première résistance de transition Rc/R1tp est compris entre 1,1 et 400,
et dans lequel un rapport de la résistance centrale à la deuxième résistance de transition Rc/R2tp est compris entre 1,1 et 400.

4. Ensemble de chauffage selon l'une quelconque des revendications précédentes, dans lequel une résistance totale Rt correspondant à la résistance électrique entre le premier point de contact (28) et le deuxième point de contact (48) est comprise entre 0,5 Ohm et 4 Ohms, dans lequel la résistance centrale Rc est supérieure à 0,5 Ohm, dans lequel la première résistance R1 et la deuxième résistance R2 sont chacune inférieures à 100 mOhm.

5. Ensemble de chauffage selon l'une quelconque des revendications précédentes, comprenant une région longitudinale centrale s'étendant du premier point de contact (28) au deuxième point de contact (48), dans lequel une résistance électrique dans la région longitudinale centrale est inférieure à une résistance électrique en dehors de la région longitudinale centrale.

6. Ensemble de chauffage selon l'une quelconque des revendications précédentes, dans lequel l'agencement de filaments plats électriquement conducteurs est une feuille perforée, avec une surface centrale de la feuille perforée comprenant une pluralité de filaments du dispositif de chauffage et avec une première et une deuxième surfaces latérales de la feuille perforée comprenant une pluralité d'ouvertures, la première et la deuxième surfaces latérales étant agencées sur les côtés opposés de la surface centrale, la première surface latérale comprenant le premier point de contact (28) et la deuxième surface latérale comprenant le deuxième point de contact (48).

7. Ensemble de chauffage selon l'une quelconque des revendications 1 à 5, dans lequel l'agencement de filaments plats électriquement conducteurs est un agencement de maille (1) comprenant une surface centrale (13) et une première et une deuxième surfaces latérales (11, 15), dans lequel une maille d'une surface centrale (13) et des mailles des première et deuxième surfaces latérales (11, 15) comprennent chacune une densité de maillage, dans lequel la densité de maillage dans la surface centrale (13) est inférieure à la densité de maillage dans chacune des première et deuxième surfaces latérales (11, 15), dans lequel les première et deuxième surfaces latérales sont agencées sur des côtés opposés de la surface centrale (13), la première surface latérale (11) comprenant le premier point de contact (28) et la deuxième surface latérale (15) comprenant le deuxième point de contact (48).

8. Ensemble de chauffage selon la revendication 7, dans lequel un gradient de densité de maillage est situé entre la première surface latérale (11) et la surface centrale (13) et entre la surface centrale (13) et la deuxième surface latérale (15).

9. Ensemble de chauffage selon l'une quelconque des revendications 7 à 8, dans lequel les mailles de la première et la deuxième surfaces latérales (11, 15) comprennent une ouverture de trame supérieure à zéro et aucune ouverture de chaîne.

10. Ensemble de chauffage selon l'une quelconque des revendications 7 à 9, dans lequel dans la direction de tissage de l'agencement de filaments, un même nombre de filaments est agencé l'un à côté de l'autre dans la surface centrale et dans les première et deuxième surfaces latérales (11, 15).

11. Ensemble de chauffage selon l'une quelconque des revendications 7 à 10, dans lequel dans la direction de tissage de l'agencement de filaments, plus de filaments sont agencés dans une région longitudinale centrale qu'en dehors de la région longitudinale centrale.

12. Ensemble de chauffage selon l'une quelconque des revendications précédentes, comprenant en outre :
un substrat comprenant une ouverture à travers le substrat,
l'agencement de filaments plats électriquement conducteurs s'étendant sur l'ouverture du substrat ; et une attache fixant l'agencement de filaments plats au substrat.

13. Ensemble de chauffage selon la revendication 12, dans lequel l'attache est électriquement conductrice et sert de contact électrique pour fournir un courant de chauffage à travers l'agencement de filaments.

14. Ensemble de chauffage selon l'une quelconque des revendications 12 à 13, dans lequel l'attache est une attache mécanique telle que des pinces, des vis ou une attache à verrouillage de forme.

15. Système de génération d'aérosol à fonctionnement électrique comprenant :
un dispositif générateur d'aérosol et une cartouche comprenant un substrat formant aérosol liquide ;
un ensemble de chauffage perméable aux fluides selon l'une quelconque des revendications 1 à 14 pour chauffer le substrat formant aérosol liquide,
dans lequel la cartouche comprend un logement ayant une ouverture, avec l'ensemble de chauffage s'étendant sur l'ouverture du logement de la cartouche,
et dans lequel le dispositif générateur d'aérosol comprend un corps principal définissant une cavité pour la réception de la cartouche, une source d'alimentation électrique et des contacts électriques pour connecter la source d'alimentation électrique à l'ensemble de chauffage.
